# EUROPEAN PATENT APPLICATION

(11) **EP 1 449 563 A1**
(43) Date of publication of application: **25.08.2004**
(21) Application number: 04250856.4
(22) Date of filing: 18.02.2004
(51) Int. Cl.: A61N 7/02

(54) **Externally-applied high intensity focused ultrasound (hifu) for therapeutic treatment**

(30) Priority: 19.02.2003 US 370381
(71) Applicant: Biosense Webster, Inc., Diamond Bar, California 91765 (US)
(72) Inventor: Govari, Assaf, Haifa 34400 (IL)
(74) Representative: Mercer, Christopher Paul

(57) **Abstract**

Apparatus for performing a therapeutic procedure using ultrasound is provided, including a beacon, adapted to be placed at a site in a body of a subject, and a set of one or more ultrasound transducers, each transducer adapted to detect a respective beacon signal coming from the beacon, and adapted to output a time-reversed ultrasound signal, reversed in time with respect to a property of at least one of the beacon signals.

## Description

### CROSS-REFERENCES TO RELATED APPLICATIONS

This application is related by subject matter to European Patent Application No. ......... entitled, "Externally-Applied High Intensity Focused Ultrasound (HIFU) For Pulmonary Vein Isolation," filed on the same date as the present Patent Application (Attorney's ref: P036735EP).

### FIELD OF THE INVENTION

The present invention relates generally to use of ultrasound in medical technology applications, and specifically to methods and apparatus for performing therapeutic procedures with high-intensity focused ultrasound (HIFU).

### BACKGROUND OF THE INVENTION

The use of ultrasound for imaging and diagnosis of disease is well known in the medical field. In medical ultrasound applications, signals are delivered to a patient by an ultrasound transducer which converts electrical signals into ultrasound pulses. The ultrasound beam is absorbed, dispersed, and reflected within the patient's body.

In diagnostic applications, reflected ultrasound energy is analyzed and processed to generate image and/or flow information about anatomical structures within the patient's body. Typical examples of such ultrasound usage are imaging the heart, evaluating the growth and condition of an unborn fetus, and measuring the size and condition of a prostate gland. Ultrasound, when applied at power levels required for imaging, has not been found to have deleterious side effects associated with other forms of radiated energy, such as X-rays, microwaves and other electromagnetic fields. Therefore, ultrasound imaging systems have distinct advantages over other imaging modalities.

In therapeutic applications, absorbed ultrasound energy is used to change the state of the target area. In particular, ultrasound energy applied at high power densities can have significant physiological effects on tissues. These effects may result from either thermal or mechanical response of the tissue to the imposition of ultrasound energy. Thermal effects include hyperthermia and ablation of tissue. The absorption of ultrasound energy at the target area induces a sudden temperature rise, which causes coagulation or ablation of target area cells. Consequently, high intensity focused ultrasound (HIFU) can be used for rapid heating of human tissue to arrest bleeding and to ablate tumors. Mechanical effects of ultrasound include the breaking up of solid objects, liquefaction of tissues, and cavitation. A typical application is the lithotriptor, which uses a large radiating surface external to the body of the patient to focus short bursts of ultrasound energy into the patient, in order to mechanically fragment kidney stones.

HIFU has been proposed as a therapeutic method for treating pathologies which manifest themselves in a localized manner. Such pathologies include, for example, neoplastic and other diseases of or in the brain, breast, liver and prostate. Although other surgical procedures have been developed for these diseases, such surgery is often lengthy, complex, and expensive. Additionally, the surgery is often repeated, to ensure that the problems have been completely eliminated. HIFU therapy, on the other hand, is a relatively simple and minimally-invasive alternative, in which the patient is potentially subjected to less trauma, thus promoting faster healing.

In therapeutic applications of ultrasound, it is important that the applied ultrasound energy causes an intended change of state solely at a target area, without adversely affecting other tissue within the patient. The effective therapeutic dose must be delivered to the target area while the thermal and mechanical effects in intermediary and surrounding tissue are minimized. Therefore, proper focusing and control of HIFU is one of the primary criteria for successful therapeutic application of ultrasound.

US Patent 6,042,556 to Beach et al. describes methods for controlling the phase of each transducer element of an external HIFU transducer array in order to compensate for phase change introduced by different tissues along a path towards a treatment site.

US Patent 5,762,066 to Law et al. describes a HIFU system consisting of an intracavity probe having two active ultrasound radiating surfaces with different focal geometries.

US Patent 6,007,499 to Martin et al. describes methods for reducing bleeding during surgery and for stemming hemorrhaging. HIFU is described as being used to form cauterized tissue regions prior to surgical incision, for example, so as to form a cauterized tissue shell around a tumor to be removed.

US Patent 5,092,336 to Fink describes a device for localization and focusing of acoustic waves in tissues. The invention is based upon a technique known as time-reversed acoustics, which is described in an article by Fink, entitled, "Time-Reversed Acoustics," *Scientific American,* November 1999, pp. 91 - 97, which is also incorporated herein by reference. Essentially, a target is enclosed by an array of transducers that delivers an unfocused acoustic beam on a reflective target in a medium, for instance, on a tumor in organic tissue. Reflected signals detected by the array of transducers are stored, the distribution in time and the shapes of the echo signals are time-reversed, and the reversed signals are applied to the respective transducers of the array. In most cases, the target constitutes a secondary source, which reflects or scatters a wave beam applied to it. The target may, for instance, consist of a kidney stone reflecting a beam received from an array of transducers, or a small tumor impregnated with a contrast agent.

US Patent 6,161,434 to Fink et al. describes methods to use time-reversed acoustics to search for a faint sound source. US Patent 5,428,999 to Fink, which is also incorporated herein by reference, describes methods for detecting and locating reflecting targets, ultrasound echographic imaging, and concentrating acoustic energy on a target.

US Patent 5,590,657 to Cain et al. describes an ultrasound system including a phased array of ultrasound transducers located outside the patient. Methods for refocusing the beam are described.

US Patent 5,769,790 to Watkins et al. describes a system for combining ultrasound therapy and imaging.

US Patent 5,366,490 to Edwards et al. describes a method for applying destructive energy to a target tissue using a catheter.

US Patent 6,128,958 to Cain describes an architecture for driving an ultrasound phased array.

US Patents 5,207,214 and 5,613,940 to Romano describe an array of reciprocal transducers which are intended to focus intense sound energy without causing extraneous tissue damage.

US Patent 5,241,962 to Iwama describes the use of ultrasonic pulses and echo signals to disintegrate a calculus.

PCT Patent Publication WO 97/29699 to Ben-Haim, entitled, "Intrabody energy focusing," describes methods for optimizing irradiation of a target area of the body by using a radiation-sensing probe inserted into the body.

US Patents 5,807,395 to Mulier et al., and 6,190,382 to Ormsby et at., describe systems for ablating body tissue using radio frequency. US Patents 6,251,109 and 6,090,084 to Hassett et al., 6,117,101 to Diederich et al., 5,938,660 and 6,235,025 to Swartz et al., 6,245,064 and 6,024,740 to Lesh et al., 6,012,457, 6,164,283, 6,305,378 and 5,971,983 to Lesh, 6,004,269 to Crowley et al., and 6,064,902 to Haissaguerre et al., describe apparatus for tissue ablation to treat atrial arrhythmia, primarily by ablating tissue located within the pulmonary veins or on the ostia of the pulmonary veins.

An article entitled, "Extracardiac ablation of the canine atrioventricular junction by use of high-intensity focused ultrasound," by SA Strickberger et al., *Circulation,* 100, 203-208 (1999) describes the experimental use of HIFU to ablate the atrioventricular junction within a beating heart.

An article entitled, "High intensity focused ultrasound phased arrays for thermal ablation of myocardium," by JU Kluiwstra et al., University of Michigan Medical Center, Department of Internal Medicine (undated) describes the experimental use of a combined ultrasound imaging and therapy system to place lesions at various locations in the heart under real-time ultrasound image guidance.

The following references may be useful:
Hill CR et al., "Review article: High intensity focused ultrasound-potential for cancer treatment," *Br J Radiol,* 68(816), 1296-1303 (1995)
Lin WL et al., "A theoretical study of cylindrical ultrasound transducers for intracavitary hyperthermia, *Int J Radiat Oncol Biol Phys,* 46(5), 1329-36 (2000)
Chapelon JY et al., "New piezoelectric transducers for therapeutic ultrasound," *Ultrasound Med Biol,* 26(1), 153-159 (2000)
Chauhan S et al., "A multiple focused probe approach for high intensity focused ultrasound based surgery," *Ultrasonics,* 39(1), 33-44 (2001)
Lee LA et al., "High intensity focused ultrasound effect on cardiac tissues: Potential for clinical application," *Echocardiography,* 17(6 Pt 1), 563-566 (2000)
Sommer FG et al., "Tissue ablation using an acoustic waveguide for high-intensity focused ultrasound," *Med Phys,* 24(4), 537-538 (1997)
Kluiwstra JU A et al., "Ultrasound phased arrays for noninvasive myocardial ablation: Initial studies," *IEEE Ultrasonics Symposium Proceedings,* Vol. 2, 1604-1608 (1995)

### SUMMARY OF THE INVENTION

It is an object of some aspects of the present invention to provide improved apparatus and methods for performing therapeutic procedures using high-intensity focused ultrasound (HIFU).

It is a further object of some aspects of the present invention to provide apparatus and methods that increase the precision of procedures using HIFU.

It is yet a further object of some aspects of the present invention to provide apparatus and methods that increase the efficiency of procedures using HIFU.

It is still a further object of some aspects of the present invention to provide apparatus and methods that increase the effectiveness of procedures using HIFU.

It is also an object of some aspects of the present invention to provide apparatus and methods that reduce the risk of procedures using HIFU.

It is an additional object of some aspects of the present invention to provide apparatus and methods that enlarge the range and variety of medical conditions that can be treated by HIFU.

In preferred embodiments of the present invention, apparatus for performing therapeutic procedures using HIFU comprises a beacon placed within the body of a patient at or in close proximity to a target location of a procedure. An array of transducers located outside the patient's body, typically on the skin, detects respective beacon signals comprising ultrasound energy from the beacon, and delivers electrical signals to a control unit, responsive to the detected energy. The control unit stores the shapes and distributions in time of the signals. Using techniques of time-reversed acoustics, the control unit reverses the distributions in time and the shapes of the signals and drives each transducer in the array to output its respective reversed signal, such that the generated waveform is accurately focused on the site of the beacon or in a vicinity thereof. The time-reversed waveforms are typically amplified, thus enabling a substantial amount of energy to be received within a short period at the target location in the vicinity of the beacon.

In some preferred embodiments of the present invention, the beacon actively emits ultrasound energy, preferably omnidirectional ultrasound energy. Alternatively, the beacon comprises a passive ultrasound reflector. In this case, the beacon is typically illuminated by a generally unfocused ultrasound signal, generated by some or all of the transducers of the array or by another source external to the body, and the array of transducers subsequently detects the echo of the illuminating signal. The beacon preferably is of a known geometry that produces a sharp and distinguishable signature that can be identified by the transducers or the control unit. Alternatively or additionally, the beacon is characterized by substantially higher reflectivity than the natural reflectivity of the surrounding tissue. Further alternatively, the beacon comprises a crystal with a predefined resonance frequency and a high Q, whereby the beacon is detected by the transducers when they generate the ultrasound signal at the resonance frequency. Still further alternatively, the beacon comprises a bubble containing an ultrasound contrast agent that reflects a known harmonic of the applied ultrasound signal. In this case, the transducers or control unit identifies the beacon by detecting the known harmonic of the applied frequency.

Thus, "beacon," as used in the context of the present patent application and in the claims, is to be understood as being indicative of both active and passive elements that respectively emit or reflect ultrasound energy.

In some preferred embodiments of the present invention, the beacon is temporarily placed in the patient, typically by affixing the beacon to the distal end of a catheter for insertion into the body. To assist in placing the beacon at a desired site, methods and apparatus are preferably but not necessarily utilized which are described in EP-A-1 321 097 and EP-A-1 321 708. Alternatively or additionally, methods and apparatus known in the art are used to facilitate the placement of the beacon at a desired site in the body.

In some preferred embodiments, the beacon is implanted in a patient, typically during a biopsy, within or adjacent to tissue of interest. If the biopsy is negative, the beacon is either removed or allowed to remain. If the biopsy is positive (e.g., indicative of a malignant tumor), the beacon is used to help focus HIFU waves utilizing the techniques described herein. Alternatively, the beacon is affixed to an implant which is subsequently implanted in the body.

In some embodiments in which the beacon comprises an active beacon, the control unit is coupled to the active beacon by leads, typically through a catheter. An electrical signal is sent to the active beacon, and the active beacon transduces the energy into ultrasound energy, which is received by the transducers outside of the patient's body. Alternatively, the active beacon comprises circuitry which wirelessly receives energy radiated from a remote site, typically located outside the patient's body, and the active beacon transduces the energy into the outputted ultrasound energy. Preferably, the energy received from the remote site comprises ultrasound energy and/or electromagnetic energy.

In some preferred embodiments of the present invention, the beacon is placed sequentially at a plurality of locations in the patient's body, in close proximity to the structure where the intended therapy is to be performed, preferably at locations generally surrounding the structure. Typically, the plurality of locations comprises at least four non-coplanar locations. Alternatively, more than one beacon is implanted in the patient, at a respective plurality of locations in the patient's body.

According to both of these alternatives, the beacon can be either an active element or a passive reflector illuminated by an ultrasound signal, as described hereinabove. Preferably, but not necessarily, each beacon comprises a position sensor, to enable a determination of its location with respect to the target structure to be heated or destroyed. Alternatively, other techniques (e.g., fluoroscopy) are utilized to facilitate the determination of the beacon's position with respect to the structure.

When the beacon comprises an active element, the beacon receives an electrical signal, either over leads or wirelessly, and the beacon transduces the energy into ultrasound energy, while the beacon is at each of the locations.

Whether the beacon comprises an active or passive element, the waveform from the beacon is detected by each of the transducers and is transformed into electrical signals, and the shapes and relative positions in time of the signals are stored in the control unit. Upon receipt of the electrical signals from the transducers for each of the beacon locations, the control unit uses techniques of time-reversed acoustics to reverse the distributions in time and the shapes of each of the signals received by each of the transducers from each of the beacon locations. In order to focus the time-reversed waveforms generated by the transducers onto the target structure rather than the beacon locations, the control unit calculates an appropriate transmission signal for each transducer. For each transducer, the control unit uses the known positions of the beacon locations and of the target structure to calculate a transmission signal that is focused on the structure rather than the beacon locations.

The control unit preferably drives each of the transducers to output its respective calculated time-reversed signal, such that the generated waveform is accurately focused on the structure, with any distortions occurring during transmission through the tissue from the vicinity of the structure to the transducers being generally compensated for by identical but time-reversed distortions on the return path. Typically, the calculated time-reversed waveforms are amplified in order to deposit substantial quantities of energy in short time periods in the immediate vicinity of the structure.

Advantageously, in these embodiments of the present invention, the beacon does not need to be inserted into the target structure. Such insertion is often difficult or impossible to perform, such as when the structure is a kidney stone.

In accordance with these preferred embodiments of the present invention, the use of a beacon positioned at the target site raises the accuracy of HIFU therapeutic procedures by increasing the probability that the HIFU waves are focused on the precise location of the targeted tissue, and, additionally, that each external transducer is focused on generally the same location of the target. This is in contrast to other methods, which attempt to focus the HIFU waves on tissue having particular reflective or absorptive characteristics.

The increased accuracy provided by these embodiments of the present invention typically increases the efficiency and/or effectiveness of procedures and reduces the likelihood of damage to untargeted surrounding tissue caused by HIFU waves. Additionally, in preferred embodiments of the present invention that utilize a beacon on the distal end of a catheter, application of HIFU from transducers located outside the body advantageously allows the use of a smaller catheter, because ablating hardware does not need to be included in the catheter.

Advantageously, the placement of the beacon enables HIFU to be accurately focused on a moving target, such as on a site in the heart. The array of transducers can be adapted to refocus the HIFU at a rapid rate responsive to the ultrasound energy from the moving beacon.

It is to be appreciated that whereas preferred embodiments of the present invention are described herein with respect to applying ultrasound energy to tissue of the patient, this is by way of illustration and not limitation, and the scope of the present invention includes utilizing the techniques described herein to apply ultrasound energy to and/or to ablate other structures within the body (e.g., kidney stones). Additionally, whereas preferred embodiments of the present invention are described herein with respect to applying ultrasound energy to a single discrete, focused target, this is by way of illustration and not limitation, and the scope of the present invention includes applying ultrasound energy to a series of discrete, focused targets, or in a continuous line or other shape in a tissue.

There is therefore provided, in accordance with a preferred embodiment of the present invention, apparatus for performing a therapeutic procedure using ultrasound, including:
a beacon, adapted to be placed at a site in a body of a subject; and
a set of one or more ultrasound transducers, each transducer adapted to detect a respective beacon signal coming from the beacon, and adapted to output a time-reversed ultrasound signal, reversed in time with respect to a property of at least one of the beacon signals.

In an embodiment, the set of transducers is adapted to be applied to an external surface of the body of the subject.

In an embodiment, each transducer is adapted to configure its time-reversed signal to be reversed in time and shape with respect to a property of the beacon signal detected by that transducer.

In an embodiment, each transducer is adapted to amplify the time-reversed signal prior to outputting the time-reversed signal.

In an embodiment, the beacon is adapted to be implanted at the site.

For some applications, the beacon is adapted to be placed in a vicinity of cancerous tissue at the site, and the set of transducers is adapted to output the time-reversed signal so as to destroy the cancerous tissue. For other applications, the beacon is adapted to be placed in a vicinity of non-cancerous tissue at the site, and the set of transducers is adapted to output the time-reversed signal so as to heat the non-cancerous tissue.

For some applications, the beacon is adapted to be placed in a vicinity of electrically-malfunctioning tissue at the site, and the set of transducers is adapted to output the time-reversed signal so as to destroy the tissue. For other applications, the beacon is adapted to be placed in a vicinity of a stone at the site, and the set of transducers is adapted to output the time-reversed signal so as to break the stone.

In an embodiment, the one or more transducers include a single ultrasound transducer, and the single ultrasound transducer is adapted to output the time-reversed signal reversed in time with respect to a shape of the beacon signal detected by the single ultrasound transducer. Alternatively, the one or more transducers include a plurality of transducers, each adapted to output the time-reversed signal reversed in time with respect to a sequence of detecting the beacon signal at the plurality of transducers.

In an embodiment, each transducer is adapted to regulate a timing parameter of the outputting of the respective time-reversed signal, responsive to a position of the transducer, a position of the beacon, and a position of a target in the body.

In an embodiment, the apparatus includes an instrument having a distal end, which is adapted to be inserted into the body and brought to the site, and the beacon is adapted to be affixed in a vicinity of the distal end of the instrument. For some applications, the instrument includes a catheter.

In an embodiment, the beacon includes a passive element, the apparatus includes an ultrasound transmitter, adapted to illuminate the beacon with an illuminating ultrasound signal, and each transducer is adapted to detect an echo signal coming from the beacon responsive to illumination by the illuminating signal, and to output the time-reversed signal, reversed in time with respect to a property of the echo signal detected by that transducer. In an embodiment, the transmitter includes one of the transducers. In an embodiment, the passive element includes an ultrasound reflector, characterized by higher ultrasound reflectivity than a natural level of ultrasound reflectivity at the site.

In an embodiment, the passive element is of a geometry that produces a distinguishable signature in the echo signal when the element is illuminated by the transmitter, and one or more of the transducers are adapted to detect the signature in the echo signal and to output the time-reversed signal responsive thereto. Alternatively or additionally, the passive element includes a crystal having a predefined resonance frequency. Further alternatively or additionally, the passive element includes an ultrasound contrast agent that reflects a known harmonic of the illuminating signal, and one or more of the transducers are adapted to detect the known harmonic and to output the time-reversed signal responsive thereto.

In an embodiment, the apparatus includes a control unit, adapted to store the beacon signals received from the beacon by each transducer, and adapted to drive each transducer to output its respective time-reversed signal. In an embodiment, each transducer is adapted to transform the beacon signals it receives into electrical signals, and to transmit the electrical signals to the control unit. For some applications, the control unit is adapted to drive each transducer to configure its respective time-reversed signal to have a greater amplitude than a corresponding amplitude of the beacon signal received by the respective transducer.

In an embodiment, the beacon includes circuitry to receive energy, and is adapted to transduce the received energy so as to generate the beacon signal. For some applications, the beacon is adapted to configure the beacon signal to include one or more generally omnidirectional pulses.

In an embodiment, the apparatus includes external power circuitry, and a set of one or more wires connecting the external power circuitry to the beacon, and the external power circuitry is adapted to transmit the energy to the beacon through the wires.

For some applications, the circuitry is adapted to receive the energy wirelessly. In an embodiment, the apparatus includes a power transmitter, adapted to be located outside the body, and to wirelessly transmit the energy to the beacon. For some applications, the power transmitter is adapted to wirelessly transmit ultrasound energy to the beacon. Alternatively, the power transmitter is adapted to wirelessly transmit electromagnetic energy to the beacon.

In an embodiment, the beacon is adapted to be placed in sequence at a plurality of locations in a vicinity of the site. In an embodiment, the beacon is adapted to be placed at the plurality of locations, such locations including at least four non-coplanar locations. In an embodiment, each transducer is adapted to detect a respective beacon signal when the beacon is at each respective location, and is adapted to subsequently output the time-reversed signal, responsive to the respective beacon signals from the beacon at each respective location and responsive to a position of a target in a vicinity of the site.

In an embodiment, the beacon includes a location sensor, adapted to generate a respective location signal responsive to a respective location of the beacon, and each transducer is adapted to output the time-reversed signal responsive to the location signals.

In an embodiment, the one or more transducers include a plurality of ultrasound transducers, adapted to output the time-reversed signals responsive to the position of the target and reversed in time with respect to a sequence of detecting, at the plurality of transducers, the beacon signal when the beacon is at each location.

In an embodiment, each transducer is adapted to regulate a timing parameter of the outputting of the time-reversed signal, responsive to a position of the transducer, a position of the beacon when the beacon is at each location, and the position of the target.

In an embodiment, the beacon includes a plurality of beacons, each adapted to be implanted at a different location in a vicinity of the site. For some applications, the plurality of beacons includes four beacons, adapted to be placed at different locations near the site, such locations including at least four non-coplanar locations.

In an embodiment, each transducer is adapted to detect a beacon signal coming from each beacon, and, subsequently, to output the time-reversed signal responsive to the beacon signals and a position of a target in a vicinity of the site. In an embodiment, each of the beacons includes a respective location sensor, adapted to generate a respective location signal responsive to a respective location of the respective beacon, and each transducer is adapted to output the time-reversed signal responsive to the location signals.

In an embodiment, the one or more transducers include a plurality of ultrasound transducers adapted to output respective time-reversed signals, responsive to the position of the target and reversed in time with respect to a sequence of detecting, at the plurality of transducers, the beacon signal coming from each beacon.

In an embodiment, each transducer is adapted to regulate a timing parameter of the outputting of the time-reversed signal, responsive to a position of the transducer, a location of each beacon, and the position of the target.

There is also provided, in accordance with a preferred embodiment of the present invention, a method for performing a therapeutic procedure using ultrasound, including:
detecting, at one or more detection locations, respective beacon signals coming from a beacon placed at a site in a body of a subject;
reversing each of the beacon signals with respect to a time-based property thereof, to obtain respective time-reversed ultrasound signals; and
generating the respective time-reversed ultrasound signals at each of the one or more locations.

### BRIEF DESCRIPTION OF THE DRAWINGS

The present invention will be more fully understood from the following detailed description of the preferred embodiments thereof, taken together with the drawings, in which:
Fig. 1 is a simplified pictorial illustration showing a HIFU therapeutic system applied to a patient, in accordance with a preferred embodiment of the present invention;
Fig. 2 is a simplified pictorial illustration showing a HIFU therapeutic system applied to a patient, in accordance with another preferred embodiment of the present invention;
Figs. 3A and 3B are timing diagrams showing one example of electrical signals respectively conveyed by the transducers to a control unit (Fig. 3A) and conveyed by the control unit to the transducers (Fig. 3B), in accordance with a preferred embodiment of the present invention;
Fig. 4A is a two-dimensional schematic diagram illustrating a simplified example of the placement of one or more beacons at a plurality of locations in a subject, in accordance with a preferred embodiment of the present invention;
Figs. 4B and 4C are timing diagrams showing electrical signals respectively conveyed by the transducers to a control unit (Fig. 4B) and determined by the control unit using techniques of time-reversed acoustics (Fig. 4C) for the example of Fig. 4A, in accordance with a preferred embodiment of the present invention; and
Fig. 5 is a simplified pictorial illustration showing a HIFU therapeutic system applied to a patient, in accordance with a preferred embodiment of the present invention.

### DETAILED DESCRIPTION OF PREFERRED EMBODIMENTS

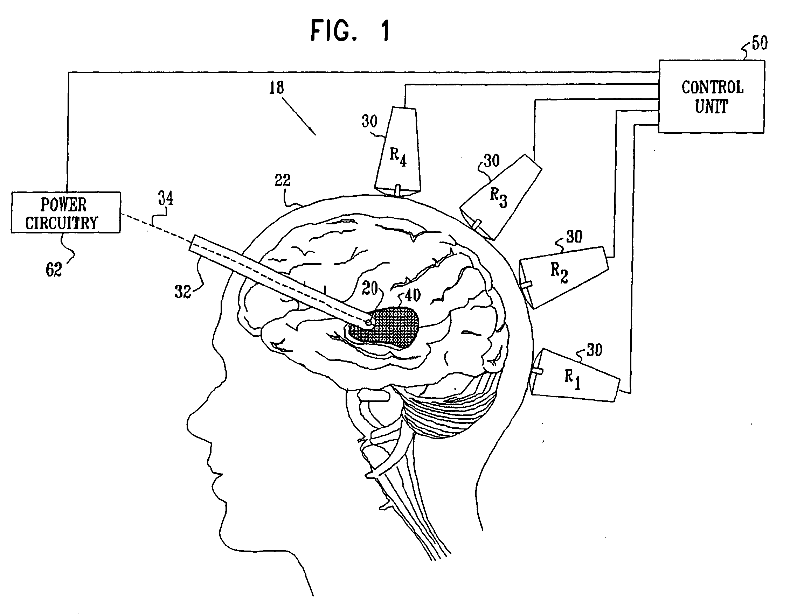

Fig. 1 is a simplified pictorial illustration showing a HIFU therapeutic system 18 applied to a patient, in accordance with a preferred embodiment of the present invention. HIFU therapeutic system 18 comprises a plurality of ultrasound transducers 30 which are coupled to a control unit 50. Transducers 30 are preferably applied in an array to a surface 22 of the patient's body such as the skin. The transducers may be of a design known in the art, and typically include piezoelectric elements. Transducers 30 detect ultrasound energy from a beacon 20, and deliver electrical signals, responsive thereto, to control unit 50.

Beacon 20 is placed in the patient's body within or in close proximity to a site at which the intended therapy is to be performed. For illustrative purposes, Fig. 1 shows beacon 20 placed at or in the vicinity of a structure 40 including a tumor within the brain. For other applications, structure 40 may include non-cancerous tissue, electrically-malfunctioning tissue, a calculus, or other structures which are amenable for treatment or ablation by the application of ultrasound.

In a preferred embodiment of the present invention, beacon 20 is temporarily placed in the patient, typically by affixing the beacon to the distal end of a guide tube 32, such as a catheter or a probe, for insertion into the body. To determine the location of the beacon, methods and apparatus are preferably utilized which are described in one or both of the above-cited EP-A-1 321 097 and EP-A-1 321 708. Alternatively or additionally, other methods known in the art, such as fluoroscopy, are appropriate for use with this embodiment.

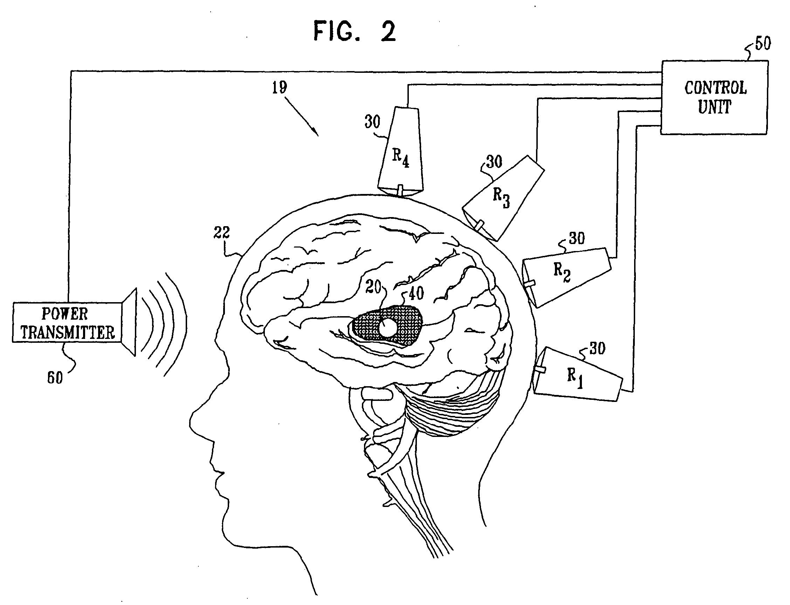

Reference is now made to Fig. 2, which is a simplified pictorial illustration showing a HIFU therapeutic system 19 applied to a patient, in accordance with a preferred embodiment of the present invention. System 19 is similar in many respects to system 18, with differences as elaborated hereinbelow. In this preferred embodiment, beacon 20 is implanted within tissue of interest in the body, typically during a biopsy. If the biopsy is negative, the beacon is either removed or allowed to remain.

For some applications, control unit 50 drives power circuitry 62 (Fig. 1) to transmit power to beacon 20 through wires 34, which typically comprise leads passed through guide tube 32. Alternatively, control unit 50 drives a power transmitter 60 (Fig. 2), typically located outside the patient's body, to wirelessly transmit power to beacon 20, in which case beacon 20 comprises circuitry which receives the radiated energy. As appropriate, the energy radiated from power transmitter 60 includes ultrasound and/or electromagnetic energy. Alternatively, one or more of transducers 30 are adapted to wirelessly transmit power to beacon 20, preferably in the form of ultrasound energy; in this embodiment, power transmitter 60 is not used. Further alternatively, beacon 20 is affixed to guide tube 32 and receives power wirelessly from power transmitter 60; in this embodiment, power circuitry 62 and power wires 34 are not used.

In accordance with a preferred embodiment of the present invention, beacon 20 transduces the received energy (whether received wirelessly or over wires 34) into outputted ultrasound energy, typically as one or more omnidirectional pulses.

In an alternative embodiment, beacon 20 comprises a passive reflector that is illuminated by an ultrasound signal, generated by a transmitter such as one or more of transducers 30, and transducers 30 detect the echo of the illuminating signal. According to this embodiment, beacon 20 can be attached to guide tube 32 (shown in Fig. 1) or implanted in the patient (as shown in Fig. 2). In this embodiment, beacon 20 preferably is of a geometry that produces a sharp and distinguishable signature that can be identified by transducers 30 or control unit 50. Alternatively or additionally, beacon 20 is characterized by substantially higher reflectivity than the natural reflectivity of structure 40 and/or the surrounding tissue. Further alternatively, beacon 20 comprises a crystal with a predefined resonance frequency and a high Q so that beacon 20 is only detected by transducers 30 when they generate the illuminating ultrasound signal at a certain frequency. Still further alternatively, beacon 20 comprises a bubble containing an ultrasound contrast agent that reflects a known harmonic of the applied ultrasound signal. In this case, transducers 30 or control unit 50 identifies beacon 20 by detecting the known harmonic of the applied frequency.

The waveform from beacon 20 is detected by each of transducers 30, typically after a delay which is dependent upon (a) the distance between the beacon and each individual transducer 30 in the array, and (b) the transmission properties of the tissue through which the ultrasound energy passes prior to being received by each individual transducer 30. In addition to the delay, the shape of the waveform received at each individual transducer 30 is typically different from that received at other transducers because of the variation in absorption properties in the various intermediate tissues. The waveform received by transducers 30 is transformed into electrical signals, and the shapes and relative positions in time of the signals are stored in control unit 50.

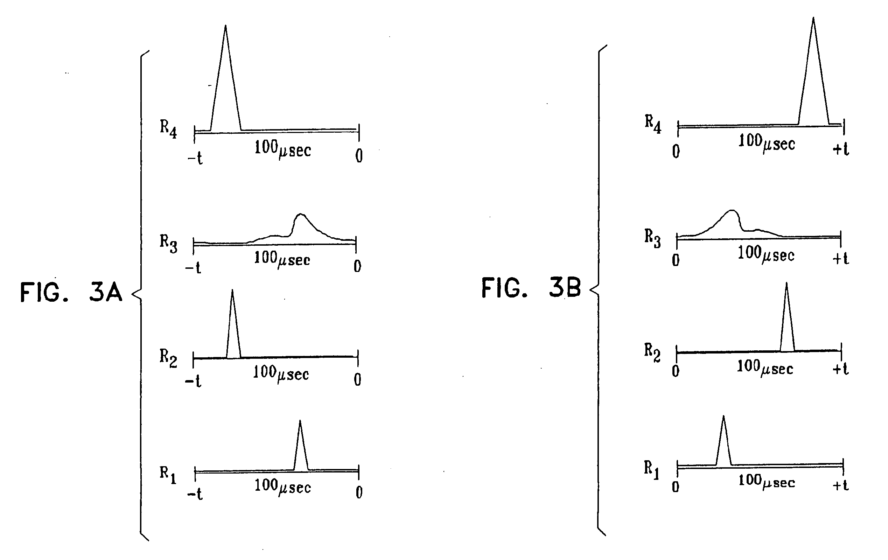

Reference is now made to Figs. 3A and 3B. Fig. 3A is a timing diagram showing one example of electrical signals conveyed from transducers 30 to control unit 50, responsive to the ultrasound signals received by the transducers from beacon 20. For illustrative purposes, transducer R₁ is shown conveying a heavily-attenuated signal approximately 40 microseconds after transducer R₄ conveys a less attenuated signal. Transducers R₂ and R₃ are shown conveying signals of different delay and shape.

In accordance with a preferred embodiment of the present invention, upon receipt of the electrical signals from transducers 30, control unit 50 uses techniques of time-reversed acoustics (e.g., those described in the above-cited patents or article by Fink, all of which are incorcorporated herein by reference) to reverse the distributions in time and the shapes of the signals.

Fig. 3B is an example timing diagram showing reversed electrical signals generated by control unit 50, corresponding to the example signals shown in Fig. 3A, in accordance with a preferred embodiment of the present invention. For some applications, control unit 50 drives each of transducers 30 to output its respective reversed signal, such that the generated waveform is accurately focused on the site of beacon 20, with any distortions occurring during transmission through the tissue from beacon 20 to transducers 30 being compensated for by generally identical but time-reversed distortions on the return path. Typically, the time-reversed waveforms are amplified in order to deposit substantial quantities of energy in short time periods in the immediate vicinity of the beacon.

For some applications, particularly when the precise shape of the generated waveforms is not expected to greatly affect the amount of heat deposited at the site of beacon 20, control unit 50 reverses the sequence of ultrasound signals received at transducers 30 in order to generate the sequence of time-reversed waveforms generated by the transducers. Thus, for example, if four transducers receive shaped pulses at respective times t = 0, 20, 45, and 50 microseconds, then the control unit may be adapted to drive the respective transducers to output square pulses at times 150, 130, 105, and 100 microseconds. These pulses converge at the site of the beacon generally simultaneously, thus resulting in a significant deposit of energy at the site in a short time period.

In a preferred embodiment of the present invention, beacon 20 is placed sequentially at a plurality of locations in the patient's body in close proximity to structure 40 (where the intended therapy is to be performed), preferably at locations generally surrounding and in close proximity to the structure, and preferably not within the structure itself. Typically, the plurality of locations comprise at least four non-coplanar locations.

Instead of or in addition to moving beacon 20 to a plurality of sites near structure 40, a plurality of beacons 20 may be implanted in the patient, at a respective plurality of locations in the patient's body. According to both of these alternatives, beacon 20 can be either an active element or a passive reflector illuminated by an ultrasound signal, as described hereinabove. Each beacon can have a different known geometry that produces a sharp and distinguished signature that can be identified by transducers 30 or control unit 50. Alternatively, each beacon comprises a crystal with a different respective predefined resonance frequency and a high Q, so that each beacon is detected by transducers 30 when they generate the ultrasound signal at the resonance frequency that is defined for each beacon. Further alternatively, each beacon comprises a bubble containing an ultrasound contrast agent that reflects a different known harmonic of the applied ultrasound signal. In this case, transducers 30 or control unit 50 identifies beacon 20 by detecting the known harmonic of the applied frequency, which is different for each beacon. Still further alternatively, all the beacons are of substantially identical construction, and control unit 50 uses algorithms, and optionally position data, to distinguish between the different beacons. Alternatively or additionally, the beacons are characterized by substantially higher reflectivity than the natural reflectivity of structure 40.

When beacon 20 comprises an active element, control unit 50 drives power circuitry 62, power transmitter 60, or one or more of transducers 30 to transmit power to beacon 20, as described hereinabove, while beacon 20 is at each of the locations. Beacon 20 transduces the received energy into outputted ultrasound energy, typically as one or more omnidirectional pulses.

Whether beacon 20 comprises an active or passive element, the waveform from beacon 20 is detected by each of transducers 30, typically after a delay dependent upon factors described hereinabove. In addition to the delay, the shape of the waveform received at each individual transducer 30 is typically different from that received at other transducers because of the variation in absorption properties in the various intermediate tissues. The waveform received by transducers 30 is transformed into electrical signals, and the shapes and relative positions in time of the signals are stored in control unit 50.

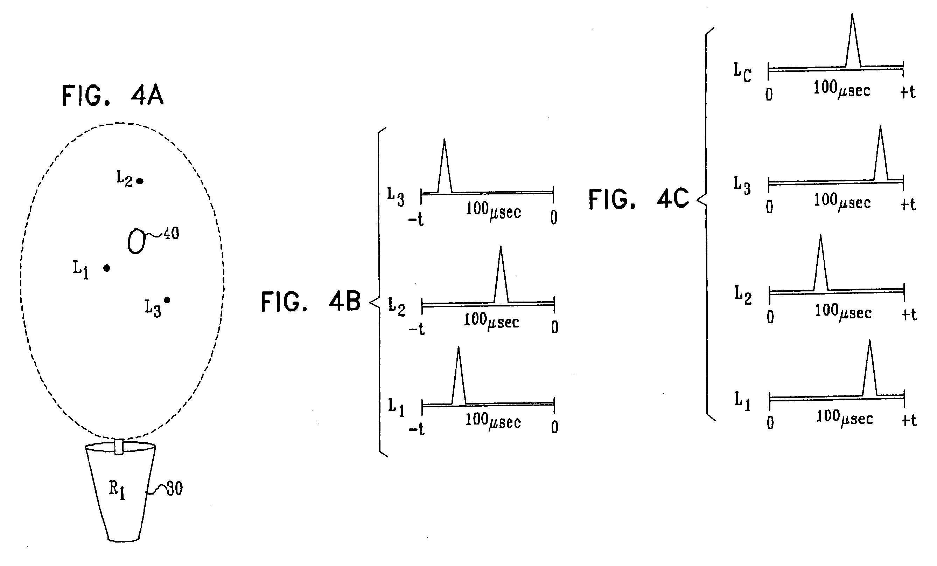

Reference is now made to Figs. 4A, 4B and 4C. Fig. 4A is a two-dimensional schematic diagram illustrating a simplified example of the placement of one or more beacons 20 at a plurality of locations in the subject, in accordance with a preferred embodiment of the present invention. Transducer R₁ is one of transducers 30, and L₁, L₂ and L₃ are three locations whereat beacon 20 or a plurality of beacons 20 have been placed surrounding structure 40. Fig. 4B is a timing diagram showing electrical signals conveyed from transducer R₁ in the example of Fig. 4A to control unit 50, responsive to the ultrasound signals received by transducer R₁ from beacon or beacons 20 while at locations L₁, L₂ and L₃. For illustrative purposes, transducer R₁ is shown conveying similarly shaped signals for each of the beacon locations, differing mainly in timing, responsive to the different distances between each of the locations and transducer R₁. For example, the peak of the waveform in signal L₂ is shown as occurring later than the peaks of the waveforms L₁ and L₃.

Upon receipt of the electrical signals from transducers 30 for each of the beacon locations, control unit 50 uses techniques of time-reversed acoustics (e.g., such as those described in the above-cited patents or article by Fink) to reverse the distributions in time and the shapes of each of the signals received by each of the transducers from each of the beacon locations. Fig. 4C is an example timing diagram showing reversed electrical signals (L₁, L₂ and L₃) determined by control unit 50, corresponding to the example signals shown in Fig. 4B, and a calculated electrical signal (L_{C}) generated by control unit 50, in accordance with a preferred embodiment of the present invention.

In order to focus the time-reversed waveforms generated by transducers 30 onto structure 40 rather than the beacon locations, control unit 50 calculates an appropriate transmission signal for each transducer, typically as follows. For each transducer 30, control unit 50 utilizes the known positions of the beacon locations and of structure 40 to calculate a transmission signal that is focused on structure 40 rather than the beacon locations. If structure 40 is within the shape defined by the beacon locations, the calculation performed preferably includes interpolation of the signals from each of the beacon locations; otherwise, the calculation typically includes extrapolation. Because the tissue in the vicinity of structure 40 is typically substantially homogenous, the calculation generally produces a signal primarily shifted in time, rather than with modified shape, relative to the signals from each of the beacon locations. Thus, if the intention of this embodiment were only to apply direct time-reversed signals, and thereby apply energy to sites L₁, L₂ and L₃, then transducer 30 in Fig. 4A would apply the lower three signals shown in Fig. 4C, in sequence and in coordination with the other transducers 30 (Fig. 1). However, in order to focus the energy on structure 40, the example calculated waveform L_{C} in Fig. 4C peaks between the peaks of L₁ and L₂, slightly closer to L₁, reflecting the relative distances of L₁ and L₂ from transducer R₁, as shown in Fig. 4A. Alternatively, the control unit performs the above-described calculations prior to time-reversing the signals, and subsequently time-reverses the calculated transmission signal.

Control unit 50 preferably drives each of transducers 50 to output its respective calculated reversed signal, such that the generated waveform is accurately focused on structure 40, with any distortions occurring during transmission through the tissue from the vicinity of structure 40 to transducers 30 being compensated for by generally identical but time-reversed distortions on the return path. Typically, the calculated time-reversed waveforms are amplified in order to deposit substantial quantities of energy in short time periods in the immediate vicinity of structure 40.

Although structure 40 has been described herein as occupying a single location, it is to be understood that structure 40 typically is a three-dimensional volume, represented in control unit 50 as a collection of three-dimensional sub-volumes. Therefore, in a preferred embodiment, control unit 50 performs the above-described calculations individually for each of the sub-volumes, and drives the transducers to focus their waveforms on each sub-volume individually.

Advantageously, in this embodiment of the present invention, beacon 20 does not need to be inserted into structure 40. Such insertion is often difficult or impossible to perform, such as when the structure is a kidney stone.

In a preferred embodiment, the position of structure 40 is not necessarily directly determined or explicitly known to control unit 50 using standard methods such as a CT scan. Rather, the locations at which beacon or beacons 20 have been placed by a physician during a medical procedure are used to delineate boundaries of a three-dimensional volume which generally surrounds structure 40. Preferably, the three-dimensional shape of structure 40 is generally determined using this procedure, and, subsequently, techniques described herein are applied to focus the time-reversed ultrasound signal from the various transducers 30 onto structure 40.

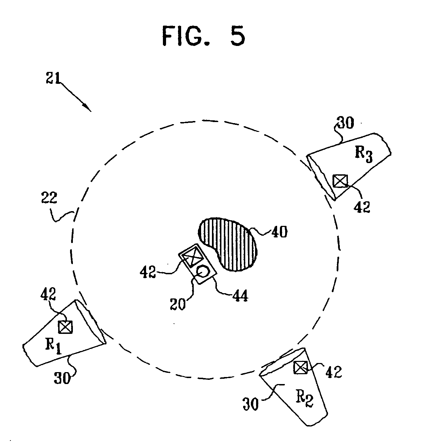

Fig. 5 is a schematic illustration of a HIFU therapeutic system 21, in accordance with a preferred embodiment of the present invention. Except for differences as described, system 21 preferably operates using techniques and apparatus similar to those described hereinabove.

According to this embodiment, each ultrasound transducer 30 preferably comprises a position sensor 42, which generates a position signal indicative of the position of the respective transducer. The position sensor 42 in one embodiment according to the present invention is an electromagnetic field responsive position sensor responsive to electromagnetic fields generated by magnetic field generators/radiators as part of a location system for determining the location, i.e. position and orientation of the position sensor 42. The location includes up to six directions and orientations such as (X, Y, Z directions and Pitch, Yaw and Roll orientations). The location system including the position sensor 42 and magnetic field generators (radiators) are described in greater detail in U.S. Patent Application Serial No. 08/793,371 entitled "Medical Diagnosis, Treatment and Imaging Systems", filed May 14, 1997, the disclosure of which is incorporated herein by reference.

A beacon package 44, comprising beacon 20, preferably also comprises a position sensor 42, which generally continuously transmits a signal indicative of the position of the beacon. Alternatively or additionally, the position of beacon 20 is determined using other methods (e.g., fluoroscopy), and the positions of the ultrasound transducers are determined using other methods (e.g., by rigid attachment to a fixed frame). Typically, the position of structure 40 is determined using imaging techniques known in the art.

Responsive to determining the generally-fixed positions of ultrasound transducers 30, beacon 20, and structure 40, control unit 50 preferably calculates a time offset to be applied to the initiation of transmission of the time-reversed signal from each transducer. Thus, in this embodiment, a plurality of beacons 20 are typically not used, and, additionally, beacon 20 is typically not moved to a number of locations in the vicinity of structure 40. Instead, the time offsets are preferably determined responsive to the fixed position of the beacon with respect to the transducers and the structure. For example, as shown illustratively in Fig. 5, transducer R₁, beacon 20, structure 40, and transducer R₃ generally lie at respective points along a line. Preferably, in this example, the time-reversed signal emitted by transducer R₃ is initiated at a time t + dt, where initiation of transmission of the time-reversed signal from transducer R₁ occurs at time t, such that the time-reversed signals from transducers R₁ and R₃ will strike structure 40 at generally the same time. Preferably, dt is selected based on the relative locations of the transducers, beacon 20, and structure 40, as well as the speed of sound in tissue. In this manner, the ultrasound energy emitted by each of the transducers is preferably focused on structure 40 in whole or in part, or, as appropriate, in sequence on sub-volumes of the structure (e.g., by scanning the focus of the time-reversed signals onto each sub-volume).

It is to be understood that each step of these embodiments of the present invention can be performed iteratively, as appropriate. For example, in some applications, control unit 50 varies the magnitude of the ultrasound energy coming from beacon 20 and/or transducers 30, responsive to various factors and conditions during a procedure. Also, particularly in the embodiments wherein the beacon comprises a passive reflective beacon, the process of focusing the transducer-generated HIFU may be iterative (e.g., as described in the above-cited patents to Fink or Fink et al. or article by Fink).

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description.
A. The present application also discloses methods for performing a therapeutic procedure using ultrasound, comprising:
   detecting, at one or more detection locations, respective beacon signals coming from a beacon placed at a site in a body of a subject;
   reversing each of the beacon signals with respect to a time-based property thereof, to obtain respective time-reversed ultrasound signals; and
   generating the respective time-reversed ultrasound signals at each of the one or more locations.
B. The application further discloses a method according to section A, wherein detecting at the one or more detection locations comprises detecting at one or more locations on an external surface of the body of the subject.
C. The application further discloses a method according to section A, wherein reversing each of the beacon signals comprises reversing each of the beacon signals with respect to the time-based property thereof and a shape-based property thereof, to obtain the respective time-reversed signals.
D. The application further discloses a method according to section A, wherein generating the time-reversed signals comprises amplifying the time-reversed signals.
E. The application further discloses a method according to section A, wherein the beacon includes a beacon implanted at the site, and wherein detecting the respective beacon signals comprises detecting the respective beacon signals coming from the beacon implanted at the site.
F. The application further discloses a method according to section A,
   wherein the beacon includes a beacon placed in a vicinity of cancerous tissue at the site,
   wherein detecting the respective beacon signals comprises detecting the respective beacon signals coming from the beacon placed in the vicinity of the cancerous tissue at the site, and
   wherein generating the time-reversed signals comprises generating the time-reversed signals so as to destroy the cancerous tissue.
G. The application further discloses a method according to section A,
   wherein the beacon includes a beacon placed in a vicinity of non-cancerous tissue at the site,
   wherein detecting the respective beacon signals comprises detecting the respective beacon signals coming from the beacon placed in the vicinity of the non-cancerous tissue at the site, and
   wherein generating the time-reversed signals comprises generating the time-reversed signals so as to heat the non-cancerous tissue.
H. The application further discloses a method according to section A,
   wherein the beacon includes a beacon placed in a vicinity of electrically-malfunctioning tissue at the site,
   wherein detecting the respective beacon signals comprises detecting the respective beacon signals coming from the beacon placed in the vicinity of the electrically-malfunctioning tissue at the site, and
   wherein generating the time-reversed signals comprises generating the time-reversed signals so as to destroy the tissue.
I. The application further discloses a method according to section A,
   wherein the beacon includes a beacon placed in a vicinity of a stone at the site,
   wherein detecting the respective beacon signals comprises detecting the respective beacon signals coming from the beacon placed in the vicinity of the stone at the site, and
   wherein generating the time-reversed signals comprises generating the time-reversed signals so as to break the stone.
J. The application further discloses a method according to section A, wherein detecting the respective beacon signals at the one or more detection locations comprises detecting a single beacon signal at a single detection location, and wherein reversing each of the beacon signals comprises reversing the single beacon signal with respect to the time-based property thereof.
K. The application further discloses a method according to section A, wherein detecting the respective beacon signals at the one or more detection locations comprises detecting a plurality of respective beacon signals at a plurality of respective detection locations, and wherein reversing each of the beacon signals comprises reversing each of the beacon signals with respect to a sequence of detecting, at the plurality of respective detection locations, the plurality of respective beacon signals.
L. The application further discloses a method according to section A, wherein generating the time-reversed signals at each of the one or more detection locations comprises regulating a timing parameter of generating the time-reversed signals at each of the one or more detection locations, responsive to a position of the respective detection location, a position of the beacon, and a position of a target in the body.
M. The application further discloses a method according to section A, wherein the beacon is affixed in a vicinity of a distal end of an instrument inserted into the body and brought to the site, and wherein detecting the respective beacon signals comprises detecting the respective beacon signals coming from the beacon when it is affixed to the instrument.
N. The application further discloses a method according to section M, wherein the beacon is affixed in a vicinity of a distal end of a catheter inserted into the body and brought to the site, and wherein detecting the respective beacon signals comprises detecting the respective beacon signals coming from the beacon when it is affixed to the catheter.
O. The application further discloses a method according to section A,
   wherein the beacon includes a passive beacon,
   wherein detecting the respective beacon signals comprises illuminating the passive beacon with an illuminating ultrasound signal, and detecting respective ultrasound echo signals coming from the passive beacon, responsive to the illuminating,
   wherein reversing each of the beacon signals comprises reversing each of the echo signals with respect to the time-based property thereof, to obtain respective reversed echo signals, and
   wherein generating the time-reversed signals comprises generating the time-reversed signals at each of the one or more detection locations, responsive to the respective reversed echo signals.
P. The application further discloses a method according to section O, wherein the passive beacon is characterized by higher ultrasound reflectivity than a natural level of ultrasound reflectivity at the site, and wherein detecting the respective echo signals comprises detecting the respective echo signals coming from the passive beacon, responsive to the higher ultrasound reflectivity.
Q. The application further discloses a method according to section O, wherein the passive beacon is of a geometry that produces a distinguishable signature in the echo signals when the beacon is illuminated with the illuminating signal, and wherein detecting the respective echo signals comprises detecting the signature in the respective echo signals.
R. The application further discloses a method according to section O, wherein the passive beacon includes a crystal having a predefined resonance frequency, and wherein detecting the respective echo signals comprises detecting the respective echo signals coming from the passive beacon, responsive to illuminating with the illuminating signal at the resonance frequency.
S. The application further discloses a method according to section O, wherein the passive beacon includes an ultrasound contrast agent that reflects a known harmonic of the illuminating signal, and wherein detecting the respective echo signals comprises detecting the known harmonic, responsive to illuminating with the illuminating signal.
T. The application further discloses a method according to section A, comprising transducing, by the beacon, energy received by the beacon so as to generate the respective beacon signals.
U. The application further discloses a method according to section T, wherein the respective beacon signals are characterized by one or more generally omnidirectional pulses, and wherein transducing the received energy comprises transducing the received energy so as to generate the generally omnidirectional pulses.
V. The application further discloses a method according to section T, comprising transmitting the energy to the beacon through a set of one or more wires connected to the beacon.
W. The application further discloses a method according to section V, comprising wirelessly transmitting the energy to the beacon.
X. The application further discloses a method according to section W, wherein the energy includes ultrasound energy, and wherein wirelessly transmitting the energy comprises wirelessly transmitting the ultrasound energy to the beacon.
Y. The application further discloses a method according to section W, wherein the energy includes electromagnetic energy, and wherein wirelessly transmitting the energy comprises wirelessly transmitting the electromagnetic energy to the beacon.
Z. The application further discloses a method according to section A, wherein detecting the respective beacon signals comprises detecting each respective beacon signal when the beacon is placed in sequence at a plurality of respective beacon locations in a vicinity of the site.
AA. The application further discloses a method according to section Z, wherein detecting the respective beacon signals comprises detecting the respective beacon signals when the beacon is placed in sequence at the plurality of beacon locations, such beacon locations including at least four non-coplanar beacon locations.
AB. The application further discloses a method according to section Z, wherein detecting the respective beacon signals comprises detecting each respective beacon signal when the beacon is at each respective beacon location, and wherein generating the time-reversed signals comprises subsequently generating the time-reversed signals, responsive to the respective beacon signals from the beacon at each beacon location and responsive to a position of a target in a vicinity of the site.
AC. The application further discloses a method according to section AB, wherein generating the time-reversed signals comprises generating the time-reversed signals responsive to a respective beacon position signal generated by the beacon at each respective beacon location.
AD. The application further discloses a method according to section AB, wherein detecting the respective beacon signals at the one or more detection locations comprises detecting a plurality of beacon signals at a respective plurality of detection locations, and wherein reversing each of the beacon signals comprises reversing each of the beacon signals responsive to the position of the target and with respect to a sequence of detecting, at the plurality of detection locations, the respective plurality of beacon signals.
AE. The application further discloses a method according to section AB, wherein generating the time-reversed signals at each of the one or more detection locations comprises regulating a timing parameter of the generation of the time-reversed signal, responsive to a position of each respective detection location, a position of the beacon when the beacon is at each beacon location, and the position of the target.
AF. The application further discloses a method according to section A, wherein detecting the respective beacon signals comprises detecting the respective beacon signals coming from a plurality of respective beacons, each implanted at a different respective beacon location in a vicinity of the site in the body.
AG. The application further discloses a method according to section AF, wherein detecting the respective beacon signals comprises detecting the respective beacon signals coming from the plurality of respective beacons implanted at the plurality of respective beacon locations, such beacon locations including at least four non-coplanar beacon locations.
AH. The application further discloses a method according to section AF, wherein generating the time-reversed signals comprises generating the time-reversed signals subsequent to and responsive to detecting the respective beacon signals from the plurality of respective beacons, and responsive to a position of a target in a vicinity of the site.
AI. The application further discloses a method according to section AH, wherein generating the time-reversed signals comprises generating the time-reversed signals responsive to location signals generated by each beacon.
AJ. The application further discloses a method according to section AH, wherein detecting the respective beacon signals at one or more detection locations comprises detecting a plurality of beacon signals at each of a plurality of detection locations, and wherein reversing each of the beacon signals comprises reversing each of the beacon signals responsive to the position of the target and with respect to a sequence of detecting, at each of the plurality of detection locations, the plurality of beacon signals coming from the respective beacons.
AK. The application further discloses a method according to section AH, wherein generating the time-reversed signals at each of the one or more detection locations comprises regulating a timing parameter of the generation of the time-reversed signals at each of the one or more detection locations, responsive to a position of the detection location, a location of each beacon, and the position of the target.

## Claims

1. Apparatus for performing a therapeutic procedure using ultrasound, comprising:
a beacon, adapted to be placed at a site in a body of a subject; and
a set of one or more ultrasound transducers, each transducer adapted to detect a respective beacon signal coming from the beacon, and adapted to output a time-reversed ultrasound signal, reversed in time with respect to a property of at least one of the beacon signals.

2. Apparatus according to claim 1, wherein the set of transducers is adapted to be applied to an external surface of the body of the subject.

3. Apparatus according to claim 1, wherein each transducer is adapted to configure its time-reversed signal to be reversed in time and shape with respect to a property of the beacon signal detected by that transducer.

4. Apparatus according to claim 1, wherein each transducer is adapted to amplify the time-reversed signal prior to outputting the time-reversed signal.

5. Apparatus according to claim 1, wherein the beacon is adapted to be implanted at the site.

6. Apparatus according to claim 1, wherein the beacon is adapted to be placed in a vicinity of cancerous tissue at the site, and wherein the set of transducers is adapted to output the time-reversed signal so as to destroy the cancerous tissue.

7. Apparatus according to claim 1, wherein the beacon is adapted to be placed in a vicinity of non-cancerous tissue at the site, and wherein the set of transducers is adapted to output the time-reversed signal so as to heat the non-cancerous tissue.

8. Apparatus according to claim 1, wherein the beacon is adapted to be placed in a vicinity of electrically-malfunctioning tissue at the site, and wherein the set of transducers is adapted to output the time-reversed signal so as to destroy the tissue.

9. Apparatus according to claim 1, wherein the beacon is adapted to be placed in a vicinity of a stone at the site, and wherein the set of transducers is adapted to output the time-reversed signal so as to break the stone.

10. Apparatus according to claim 1, wherein the one or more transducers comprise a single ultrasound transducer, and wherein the single ultrasound transducer is adapted to output the time-reversed signal reversed in time with respect to a shape of the beacon signal detected by the single ultrasound transducer.

11. Apparatus according to claim 1, wherein the one or more transducers comprise a plurality of transducers, each adapted to output the time-reversed signal reversed in time with respect to a sequence of detecting the beacon signal at the plurality of transducers.

12. Apparatus according to claim 1, wherein each transducer is adapted to regulate a timing parameter of the outputting of the respective time-reversed signal, responsive to a position of the transducer, a position of the beacon, and a position of a target in the body.

13. Apparatus according to claim 1, comprising an instrument having a distal end, which is adapted to be inserted into the body and brought to the site, wherein the beacon is adapted to be affixed in a vicinity of the distal end of the instrument.

14. Apparatus according to claim 13, wherein the instrument comprises a catheter.

15. Apparatus according to claim 1,
wherein the beacon comprises a passive element,
wherein the apparatus comprises an ultrasound transmitter, adapted to illuminate the beacon with an illuminating ultrasound signal, and
wherein each transducer is adapted to detect an echo signal coming from the beacon responsive to illumination by the illuminating signal, and to output the time-reversed signal, reversed in time with respect to a property of the echo signal detected by that transducer.

16. Apparatus according to claim 15, wherein the transmitter comprises one of the transducers.

17. Apparatus according to claim 15, wherein the passive element comprises an ultrasound reflector, **characterized by** higher ultrasound reflectivity than a natural level of ultrasound reflectivity at the site.

18. Apparatus according to claim 15, wherein the passive element is of a geometry that produces a distinguishable signature in the echo signal when the element is illuminated by the transmitter, and wherein one or more of the transducers are adapted to detect the signature in the echo signal and to output the time-reversed signal responsive thereto.

19. Apparatus according to claim 15, wherein the passive element comprises a crystal having a predefined resonance frequency.

20. Apparatus according to claim 15, wherein the passive element comprises an ultrasound contrast agent that reflects a known harmonic of the illuminating signal, and wherein one or more of the transducers are adapted to detect the known harmonic and to output the time-reversed signal responsive thereto.

21. Apparatus according to claim 1, comprising a control unit, adapted to store the beacon signals received from the beacon by each transducer, and adapted to drive each transducer to output its respective time-reversed signal.

22. Apparatus according to claim 21, wherein each transducer is adapted to transform the beacon signals it receives into electrical signals, and to transmit the electrical signals to the control unit.

23. Apparatus according to claim 21, wherein the control unit is adapted to drive each transducer to configure its respective time-reversed signal to have a greater amplitude than a corresponding amplitude of the beacon signal received by the respective transducer.

24. Apparatus according to claim 1, wherein the beacon comprises circuitry to receive energy, and wherein the beacon is adapted to transduce the received energy so as to generate the beacon signal.

25. Apparatus according to claim 24, wherein the beacon is adapted to configure the beacon signal to include one or more generally omnidirectional pulses.

26. Apparatus according to claim 24, comprising:
external power circuitry; and
a set of one or more wires connecting the external power circuitry to the beacon,
wherein the external power circuitry is adapted to transmit the energy to the beacon through the wires.

27. Apparatus according to claim 24, wherein the circuitry is adapted to receive the energy wirelessly.

28. Apparatus according to claim 27, comprising a power transmitter, adapted to be located outside the body, and to wirelessly transmit the energy to the beacon.

29. Apparatus according to claim 28, wherein the power transmitter is adapted to wirelessly transmit ultrasound energy to the beacon.

30. Apparatus according to claim 28, wherein the power transmitter is adapted to wirelessly transmit electromagnetic energy to the beacon.

31. Apparatus according to claim 1, wherein the beacon is adapted to be placed in sequence at a plurality of locations in a vicinity of the site.

32. Apparatus according to claim 31, wherein the beacon is adapted to be placed at the plurality of locations, such locations including at least four non-coplanar locations.

33. Apparatus according to claim 31, wherein each transducer is adapted to detect a respective beacon signal when the beacon is at each respective location, and to subsequently output the time-reversed signal, responsive to the respective beacon signals from the beacon at each respective location and responsive to a position of a target in a vicinity of the site.

34. Apparatus according to claim 33, wherein the beacon comprises a location sensor, adapted to generate a respective location signal responsive to a respective location of the beacon, and wherein each transducer is adapted to output the time-reversed signal responsive to the location signals.

35. Apparatus according to claim 33, wherein the one or more transducers comprise a plurality of ultrasound transducers, adapted to output the time-reversed signals responsive to the position of the target and reversed in time with respect to a sequence of detecting, at the plurality of transducers, the beacon signal when the beacon is at each location.

36. Apparatus according to claim 33, wherein each transducer is adapted to regulate a timing parameter of the outputting of the time-reversed signal, responsive to a position of the transducer, a position of the beacon when the beacon is at each location, and the position of the target.

37. Apparatus according to claim 1, wherein the beacon comprises a plurality of beacons, each adapted to be implanted at a different location in a vicinity of the site.

38. Apparatus according to claim 37, wherein the plurality of beacons comprises four beacons, adapted to be placed at different locations near the site, such locations including at least four non-coplanar locations.

39. Apparatus according to claim 37, wherein each transducer is adapted to detect a beacon signal coming from each beacon, and, subsequently, to output the time-reversed signal responsive to the beacon signals and a position of a target in a vicinity of the site.

40. Apparatus according to claim 39, wherein each of the beacons comprises a respective location sensor, adapted to generate a respective location signal responsive to a respective location of the respective beacon, and wherein each transducer is adapted to output the time-reversed signal responsive to the location signals.

41. Apparatus according to claim 39, wherein the one or more transducers comprise a plurality of ultrasound transducers adapted to output respective time-reversed signals, responsive to the position of the target and reversed in time with respect to a sequence of detecting, at the plurality of transducers, the beacon signal coming from each beacon.

42. Apparatus according to claim 39, wherein each transducer is adapted to regulate a timing parameter of the outputting of the time-reversed signal, responsive to a position of the transducer, a location of each beacon, and the position of the target.

43. Apparatus according to any one of claims 1 to 42 for use in performing a therapeutic procedure using ultrasound.
